Europäisches Patentamt

**European Patent Office**

Office européen des brevets

(11) Numéro de publication: **0 439 601 B1**

## FASCICULE DE BREVET EUROPEEN

(45) Date de publication de fascicule du brevet: **04.05.94**

(51) Int. Cl.5: **A61K 39/21**, C12N 15/49, C12N 15/48, C12N 15/62, C12N 1/21, C12N 5/10, C12Q 1/70, G01N 33/569

(21) Numéro de dépôt: **90913220.1**

(22) Date de dépôt: **17.08.90**

(86) Numéro de dépôt internationale : **PCT/FR90/00620**

(87) Numéro de publication internationale : **WO 91/02544 (07.03.91 91/06)**

(54) **COMPOSITION CONTENANT UN EPITOPE B DE LA GLYCOPROTEINE D'ENVELOPPE D'UN RETROVIRUS ET UN EPITOPE T D'UNE PROTEINE DISTINCTE DE CE RETROVIRUS.**

(30) Priorité: **18.08.89 FR 8911044**

(43) Date de publication de la demande: **07.08.91 Bulletin 91/32**

(45) Mention de la délivrance du brevet: **04.05.94 Bulletin 94/18**

(84) Etats contractants désignés: **AT BE CH DE DK ES FR GB IT LI LU NL SE**

(56) Documents cités:
EP-A- 328 390
EP-A- 0 352 060
WO-A-89/06543

INTERNATL. JOURNAL OF CANCER, vol. 40, 1989; pp. 403-407&NUM;

PROCEEDINGS OF THE NATL. ACADEMY OF SCIENCES USA, vol. 85, mars 1988; T.J. PALKER et al., pp. 1932-1936&NUM;

(73) Titulaire: **INSTITUT PASTEUR**
**28, rue du Docteur Roux**
**F-75724 Paris Cédex 15(FR)**

Titulaire: **UNIVERSITE PIERRE ET MARIE CURIE PARIS VI**
**4, place Jussieu**
**F-75230 Paris Cedex 05(FR)**

(72) Inventeur: **GIRARD, Marc**
**6, rue César-Frank**
**F-75015 Paris(FR)**
Inventeur: **GLUCKMAN, Jean-Claude**
**70, bld. Port-Royal**
**F-75005 Paris(FR)**
Inventeur: **BAHRAOUI, El, Mustapha**
**Bt K 150 HLM S.-Charles**
**Rue Lucien-Rolmer**
**F-13003 Marseille(FR)**

FEBS LETTERS, vol. 218, no. 2, 29 juin 1987, Elsevier Science Publishers B.V.; M.J.E. STERNBERG et al., pp. 231-237&NUM;

PROCEEDINGS OF THE NATL. ACADEMY OF SCIENCES USA, vol. 85, juin 1988; J. GOUD-SMIT et al., pp. 4478-4482&NUM;

ANALYTICAL BIOCHEMISTRY, vol. 161, no. 2, mars 1987, Academic Press, New York, NY (US); R.L. SHOEMAN et al., pp. 370-379&NUM;

(74) Mandataire: **Desaix, Anne et al**
**Ernest Gutmann - Yves Plasseraud S.A.**
**3, rue Chauveau-Lagarde**
**F-75008 Paris (FR)**

## Description

L'invention concerne des molécules hybrides comprenant au moins un peptide immunogène contenant un épitope B issu d'une glycoprotéine d'enveloppe d'un rétrovirus HIV, SIV pathogène, HTLV-I ou HTLV-II, et au moins un épitope T issu d'une protéine autre qu'une glycoprotéine d'enveloppe d'un rétrovirus HIV, SIV pathogène, HTLV-I ou HTLV-II, les deux protéines étant ou non issues du même rétrovirus.

L'invention concerne également des compositions vaccinantes mettant en oeuvre ces molécules.

Jusqu'à présent, les moyens mis en oeuvre pour constituer des compositions ayant des qualités vaccinantes protectrices vis-à-vis d'une infection par un rétrovirus susceptible d'induire un SIDA dans son hôte naturel ne se sont pas avérés satisfaisants. De nombreux problèmes se posent pour l'élaboration de compositions vaccinantes. Parmi les problèmes les plus importants, on peut mentionner le fait que le virus peut rester à l'état latent après l'infection pendant un moment très long, probablement à l'état d'un provirus intégré dans le génome des cellules hôtes. On peut également citer les problèmes dûs à la variabilité génétique considérable de ce virus notamment au niveau des glycoprotéines d'enveloppe de surface (env) les gp160 et gp120, au niveau de leurs protéines de structure, en particulier de la protéine gag aussi bien que des protéines non struturales telles que les protéines de régulation, par exemple les produits des gènes tat, rev, nef, vpr, vpu et/ou vpx.

Pour des commodités de langage, on désignera parfois dans la suite les protéines ou glycoprotéines par l'abréviation identifiant les gènes qui les codent respectivement.

On rappelle également que les rétrovirus HIV sont classés principalement en deux sous-types différents, HIV-1 et HIV-2 et qu'il existe à l'intérieur de chacun de ces sous-types, de nombreux variants dont certains présentent des différences de séquences pouvant dépasser 25%. Un autre problème résulte de la faculté du rétrovirus HIV d'échapper à la réponse immunitaire, par exemple en se propageant de cellule en cellule, évitant ainsi les anticorps neutralisants, ou encore en restant à l'état latent pendant de longues périodes. Par ailleurs, des anticorps dirigés contre une protéine ou une glycoprotéine d'un rétrovirus susceptible de provoquer un LAS cu un SIDA, en particulier les rétrovirus HIV ou SIV, par exemple des anticorps anti-gp120 et qui n'auraient pas de propriétés neutralisantes suffisantes pourraient favoriser la propagation du virus par l'intermédiaire de la liaison des complexes virus-IgG aux récepteurs Fc des macrophages.

De plus, une composition vaccinante efficace devrait permettre la neutralisation rapide du virus HIV, compte tenu du fait que HIV se multiplie dans les lymphocytes T4 et tue ces mêmes cellules, ces dernières étant activées par un contact avec des antigènes spécifiques et constituant donc un élément important de la réponse immunitaire.

Les auteurs de la publication EEBS Letters, vol. 218 (2), 1987 p 231-237, ont reconnu l'intérêt d'un vaccin qui comporterait des épitoges B et T. Un tel vaccin aurait des applications dans le cadre de l'infection par HIV.

Les inventeurs ont conçu une nouvelle composition susceptible d'être mise en oeuvre pour la fabrication d'un vaccin, et qui pourrait résoudre, du moins en partie, les problèmes posés ci-dessus.

L'invention concerne à cet égard une molécule hybride caractérisée en ce qu'elle comprend au moins un peptide immunogène contenant un épitope B issu d'une glycoprotéine d'enveloppe d'un rétrovirus HIV, SIV pathogène, HTLV-I ou HTLV-II, et au moins un épitope T issu d'une protéine autre qu'une glycoprotéine d'enveloppe d'un rétrovirus HIV, SIV pathogène, HTLV-I ou HTLV-II, les deux protéines étant ou non issues du même rétrovirus. Par réunion dans une même composition de ces épitopes B et T, il faut entendre association des deux épitopes dans des conditions leur permettant d'interagir ou, de préférence combinaison de ces deux épitopes notamment par couplage (liaisons covalentes) ou par toute autre voie non covalente, par exemple incorporation des deux épitopes dans une molécule ou structure recombinante commune obtenue par des techniques du génie génétique ou couplage physique par interactions hydrophobes. Pour la commodité du langage les produits de combinaison seront ci-après référencés souvent sous l'expression molécule hybride.

Il est rappelé que les "épitopes 3" correspondent à la séquence (ou l'une des séquences) d'acides aminés qui, au sein d'une protéine immunogène, intervient dans la production d'anticorps de préférence neutralisants du rétrovirus correspondant, chez l'hôte duquel cette protéine immunogène est administrée.

Les épitopes T interviennent au niveau de la stimulation du système immunitaire de l'hôte. Ils sont repérables notamment grâce à leur aptitude à induire une prolifération des lymphocytes de l'hôte à leur contact lorsque ceux-ci sont mis en culture (test de prolifération cellulaire).

L'invention vise également la mise en oeuvre de ces molécules hybrides pour la fabrication de vaccins protecteurs vis-à-vis de l'infection par un lentivirus, en particulier par un rétrovirus du type HIV, SIV pathogène ou HTLV-I ou HTLV-II de préférence vis-à-vis de l'infection par l'un quelconque des rétrovirus distincts les uns des autres, compte tenu de la variabilité soulignée plus haut, voir même de leurs appartenances respectives à plusieurs sous-types possibles. L'invention concerne encore un procédé pour la préparation desdites molécules hybrides, ou de compositions vaccinantes contenant plusieurs molécules hybrides de ce type, celles-ci se distinguant alors les unes des autres par des épitopes B, voire T correspondant respectivement à des séquences de peptides distinctes, issues elles-mêmes des susdits rétrovirus distincts.

Dans chacune des molécules hybrides selon l'invention le peptide immunogène contenant un ou plusieurs épitopes B issus de la glycoprotéine env du rétrovirus IV notamment HIV, ou HTLV-I ou HTLV-II, peut être combiné avec une séquence d'acides aminés porteuse comportant un ou plusieurs épitopes T issus d'une ou plusieurs protéines structurale ou non structurale soit du même HIV, SIV pathogène ou HTLV-I ou HTLV-II respectivement, soit d'un HIV, SIV pathogène, HTLV-I ou HTLV-II respectivement dont la glycoprotéine env croise immunologiquement avec celle du précédent.

Il doit être entendu que ce qui est dit dans ce qui suit à propos d'une "molécule hybride" déterminée est immédiatement transposable à d'autres "molécules hybrides" susceptibles d'être associées à la première dans des compositions vaccinantes du type sus-indiqué.

Par "séquence d'acides aminés" portant l'épitope T on entend soit toute partie de la protéine qui le contient et qui peut en fait comprendre jusqu'à la totalité de la protéine entière dont est issu l'épitope T, cette protéine (ou partie de protéine) pouvant alors jouer le rôle de protéine porteuse, soit un peptide beaucoup plus petit, en particulier réduit à la séquence de l'épitope T, ce peptide pouvant alors lui-même être lié par l'intermédiaire d'une liaison covalente ou non à une molécule porteuse distincte. Cette molécule porteuse (lorsqu'elle est présente) doit alors, grâce au poids moléculaire suffisant qu'elle doit alors présenter, contribuer a l'amplification de la capacité du système immunitaire de l'hôte auquel la composition est destinée à produire des anticorps protecteurs orientés contre les épitopes B, sans interférer immunologiquement avec les mécanismes immunitaires sollicités par les susdits B et T.

Dans un mode de réalisation particulièrement préféré de l'invention, les molécules hybrides répondant à la définition précédente sont caractérisées en ce que le peptide immunogène comportant l'épitope B et la séquence d'acides aminés porteuse comportant l'épitope T sont couplés chimiquement.

Une première catégorie de molécules hybrides de l'invention est caractérisée en ce que le peptide contenant l'épitope B correspond à l'épitope majeur de neutralisation de la glycoprotéine d'enveloppe de HIV-1 ou à une partie de cet épitope, suffisante pour conserver les propriétés d'un "épitope B" tel que définies ci-dessus. Une deuxième catégorie de molécules hybrides comprend un peptide contenant l'épitope B correspondant à l'épitope majeur de neutralisation d'un autre rétrovirus HIV ou SIV, HTLV-I ou HTLV-II. De manière avantageuse les molécules hybrides ci-dessus contiennent plusieurs épitopes B correspondant à l'épitope majeur de neutralisation.

L'épitope majeur de neutralisation en particulier de HIV-1 est issu d'une séquence peptidique, comportant d'environ 20 à 30 acides aminés, de la séquence localisée dans la boucle que forme cet épitope majeur dans une région hypervariable de la glycoprotéine d'enveloppe du rétrovirus HIV-1. Cet épitope majeur de neutralisation de HIV-1 a été décrit par PUTNEY SD et al. 1986 (Science 234, 1392-1395) et par RUSCHE JR et al. 1988 (Proc. Natl. Acad. Sci, USA, 85, 3198-3202). Cet épitope majeur de neutralisation est parfois désigné par "peptide de Putney". Il s'agit notamment de la séquence s'étendant entre approximativement le 301eme et le 336eme de la séquence d'acides aminés de la glycoprotéine d'enveloppe du rétrovirus HIV-1 Bru tel que décrit dans l'ouvrage Human Retroviruses and AIDS 1989 Myers, Rabson, Josephs, Smith, Berzofsky, WongStaal Edition "Los Alamos National Laboratory".

Entrent également dans le champ de l'invention, pour la formation des molécules hybrides, les peptides contenant un épitope B correspondant à l'épitope majeur de neutralisation de la glycoprotéine d'enveloppe d'un autre variant de HIV-1 ou encore d'un autre rétrovirus HIV, par exemple HIV-2, ou encore d'un virus présentant une parenté immunologique avec un rétrovirus HIV, par exemple le virus SIV ou d'un autre lentivirus tel que HTLV-I ou HTLV-II. Ces peptides peuvent être obtenus en prenant la séquence d'acides aminés du variant ou du rétrovirus choisi correspondant aux séquences ci-dessus définies.

Selon un mode de réalisatior avantageux de l'invention, des molécules hybrides particulières de l'invention peuvent comporter, pour former le peptide porteur de l'épitope B, les régions peptidiques de la glycoprotéine d'enveloppe de HIV-1 BRU comprenant notamment les acides aminés 267 et 128. La longueur de ces régions peptidiques est déterminée en fonction de la capacité de la région peptidique ainsi déterminée, de participer à la formation d'un épitope majeur de neutralisation fonctionnel.

L'invention vise aussi les régions peptidiques correspondantes d'un variant différent de HIV-1 BRU, ou les régions peptidiques d'un autre rétrovirus, HIV ou d'un autre lentivirus apparenté intervenant dans la constitution de l'épitope majeur de neutralisation.

4

Les molécules hybrides de l'invention, ont la propriété intéressante d'être susceptibles d'induire une bonne réponse immunitaire impliquant les cellules T et elles seraient capables de déclencher une bonne réponse immune primaire. En outre, lorsque le sujet vacciné serait ultérieurement en contact avec le virus, l'effet de rappel pourrait se déclencher par l'intermédiaire des cellules T à mémoire capables de reconnaître les déterminants T des antigènes du virus.

Dans ce qui suit on a recours à la nomenclature de désignation à une lettre des acides aminés. Il est rappelé qu'un peptide contenant l'épitope B comprend notamment une partie de l'épitope majeur de neutralisation de la glycoprotéine <u>env</u> de HIV ou de SIV, suffisante pour induire ou participer à une réponse immunitaire protectrice.

Les séquences identifiées ci-après en utilisant le code à une lettre dont les correspondances avec les acides aminés sont données ci-après :

| Alanine | A |
|---|---|
| Arginine | R |
| Asparagine | N |
| Acide Aspartique | D |
| Cysteine | C |
| Glutamine | Q |
| Acide Glutamique | E |
| Glycine | G |
| Histidine | H |
| Isoleucine | I |
| Leucine | L |
| Lysine | K |
| Methionine | M |
| Phenylalanine | F |
| Proline | P |
| Sérine | S |
| Thréonine | T |
| Tryptophane | W |
| Tyrosine | Y |
| Valine | V |

De façon particulièrement avantageuse, une molécule hybride selon l'invention est caractérisée en ce que le peptide contenant l'épitope B comprend au moins l'une des séquences suivantes ou une partie de ces séquences comprenant l'épitope B:

```
NTRKR  IRIQRGPGRA  FVTIGK-IGN

NTRKS  IRIQRGPGRA  FVTIGK-IGN

NTRKK  IRIQRGPGRA  FVTIGK-IGN

NTRGS  IRIQRGPGRA  FVTIGK-IGN

NTRKS  IYI--GPGRA  FHTTGRIIGD

NVRRS  LSI--GPGRA  FRTRE-IIGI

NTRRG  IHF--GPGQA  LYTTGIV-GD

NTRQR  TPI--GLGQS  LYTTRSR-SI

NTRKS  ITK--GPGRV  IYATGQIIGD

NTRKR  ITM--GPGRV  YYTTGQIIGD

DKRQS  TPI--GLGQA  LYTTRGRTKI
```

5

```
DKKIR QSIRIGPGKV FYAKGG---I
NTKKG IAI--GPGRT LYAREKIIGD
HTRKR VTL--GPGRV WYTTGEILGN
NTRRG SHF--GPGQA LYTTGIVGDI
KITSRQTPI--GLGQA LYTTRIKGDI
NVRRR HIHI-GPGRA FYTGEIRNI
NTRQS TPI--GLGQA LYTTRTKSI
NTTRS IHI--GPGRA FYATGDIIGTI
NKRKR IHI--GPGRA FYTTKNIIGDI
```

Dans les séquences des peptides qui précèdent et dans celles qui suivent, les tirets (lorsqu'ils sont présents) correspondent à des liaisons directes. Leur présence tient à la mise en alignement vertical des séquences témoignant d'une homologie au moins partielle dans les différents rétrovirus du type HIV ou SIV dont lesdits peptides sont issus.

Une autre molécule hybride particulièrement avantageuse est caractérisée en ce que le peptide porteur de l'épitope B comprend au moins l'une des séquences suivantes, ou une partie de ces séquences comprenant l'épitope B:

```
TRPNNNTRKR IRIQRGPGRA FVTIGK-IGN M-RQAH
TRPNNNTRKS IRIQRGPGRA FVTIGK-IGN M-RQAH
TRPNNNTRKK IRIQRGPGRA FVTIGK-IGN M-RQAH
TRPNNNTRGS IRIQRGPGRA FVTIGK-IGN M-RQAH
TRPNNNTRKS IYI--GPGRA FHTTGRIIGD -IRKAH
TRPYNNVRRS LSI--GPGRA FRTRE-IIGI -IRQAH
TRPGNNTRRG IHF--GPGQA LYTTGIV-GD -I.RAY
ARPYQNTRQR TPI--GLGQS LYTTRSR-SI -IGCAH
TRPNNNTRKS ITK--GPGRV IYATGQIIGD -IRKAH
TRPINNNTRKR ITM--GPGRV YYTTGQIIGD -IRRAH
TRPGSDKRQS TPI--GLGQA LYTTRGRTKI -IGQAH
TRPGSDKKIR QSIRIGPGKV FYAKGG---I -TGQAH
TRPNNNTKKG IAI--GPGRT LYAREKIIGD -IRQAH
TRPNNHTRKR VTL--GPGRV WYTTGEILGN -IRQAH


TRPGNNTRRG SHF--GPGQA LYTTGIVGDI -RRAY
TRPDNKITSRQ-TPI-GLGQA LYTTRIKGDI -RQAY
TRPNNNVRRR-HIHI-GPGRA FYTGEIRNI  -RQAH
TRPYKNTRQS-TPI--GLGQA LYTTRTKSI  -GQAH
TRPNNNTTRS-IHI--GPGRA FYATGDIIGTIRQAH
TRPNYNKRKR-IHI--GPGRA FYTTKNIIGDIRQAH
```

Des compositions immunogènes préférées comportent une pluralité de molécules hybrides conformes à l'invention se distinguant les unes des autres au niveau de leurs épitopes B, voire au niveau de l'ensemble de leurs épitopes respectifs, de préférence une telle composition comprend l'ensemble des séquences décrites ci-dessus ou une combinaison de plusieurs de ces séquences.

Pour le couplage des peptides définis ci-dessus avec les molécules porteuses, on aura avantageusement recours à un acide aminé tel que la cystéine ou la tyrosine placé en début ou en fin du peptide que l'on veut coupler.

Des compositions préférées contiennent la totalité ou presque des hybrides comportant respectivement les séquences épitopiques B faisant l'objet de la liste susdite.

D'autres compositions préférées contiennent des hybrides comportant les séquences épitopiques B ci-dessus combinées avec les régions peptidiques correspondant aux parties de l'épitope majeur comprenant les acides aminés 267 et 128, pour HIV-1 BRU ou des régions peptidiques avant la même fonction dans un autre lentivirus tel que HIV-2, SIV, HTLV-I, HTLV-II.

D'autres compositions d'épitopes avantageuses contiennent un épitope T caractéristique d'un virus déterminé en combinaison avec les épitopes B de ce virus et de ses variants.

D'autres molécules hybrides répondant à la définition générale donnée précédemment sont caractérisées en ce qu'elles comprennent au moins un épitope mineur de neutralisation, en particulier un épitope issu d'une région conservée de la glycoprotéine d'enveloppe de HIV ou de SIV.

On appelle épitope mineur de neutralisation, des séquences peptidiques appartenant à la glycoprotéine d'enveloppe d'un rétrovirus HIV ou SIV ou d'un autre virus cité plus haut, caractérisées en ce qu'elles appartiennent à une région conservée de la glycoprotéine env , qu'elles contiennent un épitope B et qu'elles induisent des anticorps neutralisants lorsqu'on les inocule à l'animal.

Une molécule hybride contenant un tel épitope mineur de neutralisation associé ou combiné à au moins un épitope T dans les conditions sus-indiquées a donc vocation à induire in vivo des anticorps susceptibles de neutraliser plusieurs variants ou souches de virus distincts, voire même à neutraliser plusieurs rétrovirus distincts.

De façon particulièrement préférée, les molécules hybrides contenant ces épitopes mineurs de neutralisation sont associés à une ou plusieurs molécules hybrides contenant un ou de préférence plusieurs des épitopes majeurs de neutralisation tels que décrits précédemment, voire l'ensemble de ces dernières molécules hybrides.

On entend par région conservée, un domaine de la glycoprotéine d'enveloppe, composé d'acides aminés ayant un taux de conservation d'au moins environ 85% entre les différentes souches de HIV.

Dans un mode de réalisation particulièrement préfuré des molécules hybrides de l'invention, l'épitope mineur comprend à titre d'exemple l'une des séquences suivantes, respectivement décrites par Chanh et al dans The EMBO Journal vol 5, n° 11, p.3065-3071, 1986 pour les deux premières et par HO et al dans Science vol 239 26 Février 1988 p.1021-1023 pour les deux dernières:

```
YDRPEGIEEEGGERDRDRSG

VAPTKAKRRVVQREKRAVGIGALFLGFLGAG

CTHGIRPVVSTQLLLNGSLAE

STQLLLNGSLAEEEVVIRC
```

La séquence d'acides aminés comportant l'épitope T entrant dans la composition des molécules hybrides selon l'invention est issue de la protéine codée par le gène nef ("protéine nef") ou d'une protéine gag, voire d'une protéine choisie parmi celles qui sont codées par les gènes tat, rev, vif, pol, vpr, vpu, vpx. Elle comprend avantageusement de 6 à 15 acides aminés. La nomenclature des gènes codés par HIV et SIV est décrite dans "Nature" vol 333-9 Juin 1988 Gallo et al.

Selon un mode de réalisation particulièrement préféré des molécules hybrides, la séquence d'acides aminés porteuse est issue de la protéine codée par le gène nef ou d'un antigène dérivé.

La protéine codée par le gène nef est une protéine de régulation, qui présente une immunogénicité pour les lymphocytes T auxiliaires et cytotoxiques (CTL). Cette protéine non structurale est absente des virions HIV infectieux et peut être détectée dans le cytoplasme des cellules infectées par HIV. La détection de l'expression à la surface cellulaire de la protéine nef, soit par immunofluorescence, soit par d'autres méthodes sérologiques s'avère difficile. La protéine nef subit vraisemblablement une maturation dans le cytoplasme de la cellule productrice et est exportée à la surface cellulaire, sous la forme de fragments d'oligopeptides reconnus par les lymphocytes T cytotoxiques. Les lymphocytes T cytotoxiques spécifiques du rétrovirus humain HIV comprennent plusieurs sous-populations dont une spécifique de la protéine nef. La protéine nef a été décrite dans l'article de Guy et al. Nature 1987, 330:266 et dans la demande de brevet EP.0253693 (87.401.388.6 du 15/6/87).

Par antigène dérivé, on entend toute molécule résultant d'une modification de la protéine d'origine ou d'une coupure de cette protéine, qui n'altère pas l'épitope T qu'elle contient.

D'une façon avantageuse, la séquence d'acides aminés portant un épitope T peut être choisie parmi le groupe des peptides suivants issus de la protéine nef (protéine F) de LAV-BRU (HIV-1 BRU) ou encore comprend tout ou partie des séquences d'acides aminés de ces peptides, par exemple :

PF 16 peptide de la protéine F constitué de 16 acides aminés (résidus 171-205):

GMDDPEREVLEWRFDSRLAFHHVARELHPEYFKNC

PF 17 (résidus 141-205):

CYKLVPVEPDKVEEANKGENTSLLHPVSLHGMDDP
EREVLEWRFDSRLAFHHVARELHPEYFKNC

PF 63 (résidus 185-205):

DSRLAFHHVARELHPEYFKNC

Un autre groupe de peptides porteur d'un épitope T comprend les peptides nef suivants caractéristiques de LAV-BRU.

PF 15 (résidus 118-167):

CGYFPDWQNYTPGPGVRYPLTFGWCYKLVPVEPDK
VEEANKGENTSLLHPV

PF 18 (résidus 93-122):

CKGGLEGLIHSQRRQDILDLWIYHTQGYFPD

L'invention s'applique de même aux séquences correspondantes des autres isolats de HIV-1 et des autres rétrovirus HIV ou SIV.

D'autres séquences d'acides aminés comportant un épitope T peuvent constituer des séquences porteuses pour réaliser les molécules hybrides. On peut par exemple avantageusement utiliser une protéine gag, notamment une protéine P55, P25, P18 ou P12 de HIV-1, ou encore une protéine codée par le gène gag de HIV-2 ou SIV, ou un fragment de ces protéines codées par le gène gag, dès lors qu'elles portent un épitope T, ou une préparation composite de plusieurs de ces protéines et/ou fragments.

D'autres protéines que les protéines spécifiques des rétrovirus HIV ou des rétrovirus apparentés tels que SIV, HTLV-I, HTLV-II peuvent encore intervenir dans la production de molécules hybrides conformes à l'invention en tant que molécules porteuses des épitopes B et T susdits. A cet égard on aura recours de façon avantageuse à certains antigènes constitutifs des particules du virus de l'hépatite B, par exemple les antigènes HBs, ou HBc, l'anatoxine tétanique ou encore l'hémocyanine (KLH) ou l'albumine humaine (HSA) etc... Les molécules porteuses citées ci-dessus représentent uniquement des exemples préférés parmi d'autres choix possibles. Un critère pour déterminer la possibilité d'utiliser une autre molécule est par exemple, sa compatibilité avec l'haplotype HLA du receveur de la composition vaccinante, comprenant lesdites molécules hybrides.

Des séquences d'acides aminés entrant dans la constitution des molécules hybrides peuvent donc également résulter du couplage chimique d'un peptide comportant un épitope T issu d'une protéine codée par l'un des gènes tat, rev, vif, pol, vpr, vpx, vpu, gag ou nef ou d'un antigène dérivé, sur la molécule porteuse, notamment l'antigène HBs, l'antigène HBc, l'anatoxine tétanique, l'hémocyanine, l'albumine humaine.

L'élaboration des molécules hybrides de l'invention à l'aide d'une séquence contenant au moins un èpitope B et d'une séquence porteuse comportant au moins un épitope T, peut être réalisée par couplage de ces séquences. Des molécules hybrides peuvent également être constituées par une association dans une même composition, des différentes séquences décrites précédemment.

Elles peuvent également résulter de l'expression d'un ADN recombinant comprenant à la fois une séquence nucléotidique codant pour un peptide immunogène contenant un épitope B et issu de la glycoprotéine _env_ issu d'un HIV, SIV pathogène, d'un HTLV-I ou d'un HTLV-II, et une séquence codant pour une séquence d'acides aminés, comportant au moins un épitope T tel que ci-dessus défini, le cas échéant sous le contrôle d'un promoteur permettant l'expression dans un hôte déterminé choisi par exemple parmi les bactéries, les levures, les virus ou les cellules eucaryotes.

Les peptides utilisés pour constituer les molécules hybrides de l'invention peuvent être préparés par les techniques classiques, dans le domaine de la synthèse des peptides. Cette synthèse peut être réalisée en solution homogène ou en phase solide.

Par exemple on aura recours à la technique de synthèse en solution homogène décrite par HOUBENWEYL dans l'ouvrage intitulé "Methode der Organischen Chemie" (Méthode de la chimie organique) édité par E.Wünsch, vol. 15-I et II. THIEME, Stuttgart 1974.

L'invention vise par ailleurs un procédé pour l'obtention de molécules hybrides décrites ci-dessus, dans lesquelles les séquences contenant les épitopes B et les séquences comprenant les épitopes T sont couplées chimiquement, ce procédé comprenant notamment la réaction de séquences d'acides aminés comportant l'épitope T avec un ou plusieurs peptides portant l'épitope B en présence d'un agent de couplage choisi selon les fonctions réactives mises en jeu dans ce couplage, les autres fonctions réactives éventuellement présentes ayant au besoin été protégées, puis la récupération, après déprotection éventuelle des fonctions protégées de produits de couplage obtenus comportant alors lesdits épitopes B et T.

Les systèmes de couplages permettant de réaliser la liaison entre les différentes séquences d'acides aminés seront choisis en fonction de la nature des résidus terminaux desdites séquences d'acides aminés. A titre d'exemple, lorsque les résidus terminaux sont des cystéines, le couplage avec des résidus lysines peut être réalisé en utilisant le SPDP (N-succinimidyl-3-(2-pyridylthio)-propionate, ou le sulfo MBS décrit par Lerner et al dans (Nature Octobre 1980, p801 à 805 vol.287 ) ou d'autres réactifs bifonctionnels.

Lorsque le couplage met en jeu des résidus tyrosine, d'autres réactifs de couplage peuvent être utilisés. Lorsque les résidus à coupler sont des résidus lysine, on pourra utiliser le glutaraldéhyde.

Pour fabriquer des molécules hybrides conformes à l'invention, ce procédé consistant à faire réagir un premier peptide comprenant une première fonction réactive avec un second peptide (ou plusieurs peptides) pourvu d'une seconde fonction réactive (ou de plusieurs fonctions réactives) complémentaires de celle du premier peptide, les fontions réactives distinctes éventuellement présentes sur certains de ces peptides et n'intervenant pas dans la réaction (ou les réactions) étant si besoin protégées. Il est entendu que dans ce qui précède "complémentarité" signifie la capacité des fonctions réactives de deux peptides distincts de conduire à une réaction de couplage covalent, directe ou indirecte, si besoin en présence d'un agent également bifonctionnel ou simplement d'un agent chimique favorisant la réaction de couplage covalent.

De préférence, le premier peptide comprend au moins une fonction amine, carboxyle libre, ester ou alcool susceptible d'être mise en jeu dans la réaction de couplage avec une fonction correspondante portée par le second peptide.

Conformément à une première variante du procédé selon l'invention on réalise la condensation entre un premier peptide portant au moins une fonction amine libre et un second peptide portant également au moins une fonction amine libre et l'on procède à la condensation de ces deux peptides, en présence d'un réactif difonctionnel tel que, par exemple, le glutaraldéhyde ou la benzoquinone.

Selon une autre variante du procédé, de l'invention, on fait réagir le premier peptide portant une fonction réactive amine, carboxyle, hydroxyle ou sulfhydryle selon l'une des techniques couramment utilisées en synthèse peptidique, avec le second peptide qui porte alors lui-même une fonction réactive complémentaire amine, carboxyle, hydroxyle, alcool ou sulfhydryle.

Lorsque l'on réalise le couplage entre une fonction carboxyle portée par l'un des partenaires avec une fonction amine portée par l'autre partenaire de la réaction, on réalise avantageusement la réaction en phase aqueuse en présence d'un carbodiimide hydrosoluble ou d'un carbodiimide liposoluble au sein d'un solvant organique inerte, tel que le diméthylformamide (DMF) ou le tétrahydrofuranne (THF), acétate d'éthyle, chlorure de méthylène, etc...

Des carbodiimides hydrosolubles avantageux sont constitués par le N-cyclohexyl-N' (bêta-(N-méthyl-morpholino)-éthyl)-carbodiimide p-toluène sulfonate, le chlorhydrate de (3-méthyl)-aminopropyl-carbodiimide.

Des carbodiimides appropriés pour des réactions au sein d'un solvant organique sont par exemple constitués par le N,N'-dicyclohexyl-carbodiimide.

Selon une autre variante du procédé selon l'invention, on fait réagir la fonction carboxyle portée par l'un des partenaires avec un chlorocarbonate d'alkyle (C$_2$ à C$_4$), puis l'on fait agir l'anhydride mixte obtenu à partir de ce premier partenaire avec le second partenaire portant la fonction amine devant intervenir dans le couplage au sein d'un solvant organique inerte, tel que ci-dessus défini, ou d'une phase aqueuse.

La première étape de la réaction est effectuée par exemple dans le diméthylformamide, en présence d'une amine tertiaire, par exemple la N-méthyl-morpholine, en utilisant par exemple le chlorocarbonate d'isobutyle. Après trois minutes à -16° C, on ajoute au mélange réactionnel le partenaire porteur d'une fonction amine déprotonée, par exemple avec de la N-méthyl-morpholine.

Selon une troisième variante du procédé selon l'invention, on fait réagir un ester activé préalablement formé de celui des partenaires portant le groupe carboxylique devant intervenir dans la réaction de couplage, avec l'autre partenaire portant une fonction amine. Des esters activés avantageux sont l'ester p-nitrophénylique ou l'ester N-hydroxysuccinimidique.

Ces réactions sont conduites en cas de besoin dans un solvant organique inerte, tel que le diméthyl-formamide.

Selon une autre variante encore du procédé selon l'invention, on fait réagir l'un des partenaires portant une fonction sulfhydryle avec l'autre partenaire, lequel porte alors un groupe maléimide.

On peut encore faire réagir avec le partenaire portant le groupe sulfhydryle l'autre partenaire portant un groupe 6-maléimido-caproïque-acide ou un groupe 3-(2-pyridyl-dithio)propionate.

Les fonctions réactives ne devant pas intervenir dans la réaction peuvent être protégées par des groupes de blocage classiques.

Les groupes carboxyliques peuvent notamment être protégés par des groupes benzylique, benzylidène ou anizilidène.

Ces groupes esters peuvent ensuite être éliminés notamment par hydrogénolyse. Celle-ci permet d'ailleurs également l'élimination simultanée d'autres groupes protecteurs tels que les groupes N-carbobenzoxy.

Les fonctions amine sont avantageusement protégées par des groupes benzyl-oxycarbonyle, t-butyl-oxycarbonyle ou toluène sulfonyle. Ces groupes peuvent ensuite être éliminés par hydrogénation catalytique ménagée, par exemple en présence de palladium, ou par acidolyse ou encore par action du sodium dans l'ammoniac liquide.

Les fonctions sulfhydryle n'intervenant pas dans la réaction de couplage peuvent être protégées par des groupes acétamidométhyle ou formamidométhyle.

Bien entendu, on peut avoir recours à tout autre type de liaison, mettant en jeu les combinaisons pouvant intervenir entre une fonction sulfhydrile portée par l'un des partenaires et un groupe maléimide porté par l'autre partenaire, par exemple en faisant usage de la technique décrite par T.KITAGAWA et T.AIKAGAWA dans "J. Biochem." 79 (1976), 233.

La conjugaison peut également se faire en utilisant les modes classiques de diazotisation ou de réaction mettant en jeu un isothiocyanate, notamment lorsque l'un des peptides porte une fonction aromatique aminée, et lorsque l'autre peptide porte une fonction amine susceptible d'intervenir dans cette réaction. De telles techniques de réalisation sont décrites par exemple par B.F. ERLANGER dans "Pharmcol. Rev.", 25 5 1973, p. 271.

Le couplage peut également être réalisé par la réduction d'une base de Schiff réalisée entre une fonction aldéhyde portée par l'un des peptides et une fonction amine portée par l'autre peptide (par exemple selon la technique de G.R. GRAY, "Arch. Biochem. Biophys.", 163 (1974), p. 425).

Toutes ces réactions sont en soi bien connues et le spécialiste appréciera que de nombreuses variantes peuvent être aménagées pour aboutir aux mêmes résultats. On remarquera aussi que les conjugaisons entre peptides peuvent s'effectuer au moyen d'un pontage, l'agent de pontage consistant par exemple en un réactif bifonctionnel. En l'occurrence, le groupe de pontage entre deux peptides dans le conjugué final de préférence n'excèdera pas la longueur d'un enchaînement correspondant à celle d'une chaîne hydrocarbonée p-décylique.

De tels agents de pontage sont par exemple mentionnés dans la demande de brevet FR-A-2 428 050.

Dans toutes les réactions qui précèdent, les proportions relatives des peptides mis en jeu peuvent varier selon les proportions finales désirées de chaque peptide dans la molécule hybride finale. En particulier, ces proportions relatives seront ajustées en fonction du nombre de groupes fonctionnels portés par chacun d'eux et susceptibles d'entrer dans la réaction de conjugaison avec des groupes fonctionnels complémentaires.

Les molécules hybrides selon l'invention peuvent aussi être produites par génie génétique, par l'expression dans un hôte cellulaire approprié, d'un ADN recombinant contenant à la fois au moins une séquence nucléique codant pour un peptide immunogène issu de la glycoprotéine env et portant l'épitope B et une séquence nucléique codant pour la séquence d'acides aminés portant l' épitope T le tout sous le contrôle des éléments de régulation permettant l'expression dans un hôte compétent choisi, par exemple une levure, une souche de bactéries ou une lignée de cellules eucaryotes et en récupérant et purifiant ensuite les produits d'expression obtenus.

L'invention vise aussi une composition de vaccin comprenant une pluralité de molécules hybrides de l'invention. Des compositions préférées contiennent une association de molécules hybrides correspondant à la totalité des molécules hybrides comportant les séquences d'acides aminés particulières données pour les épitopes B.

L'invention vise aussi des compositions immunogènes capables d'induire in vivo la production d'anticorps protecteurs et/ou neutralisants vis-à-vis d'un rétrovirus IV pathogène en particulier d'un HIV pathogène ou encore d'un HTLV-I ou d'un HTLV-II, caractérisées en ce qu'elles comprennent, en tant que principe actif, les molécules hybrides de l'invention, en association avec un véhicule pharmaceutique acceptable.

Les compositions immunogènes de l'invention sont avantageusement administrées sous forme de mélanges contenant le principe actif avec un adjuvant immunitaire.

Elles sont administrables par exemple par injection, par voie parentérale.

Avantageusement, les compositions immunogènes selon l'invention contiennent en outre un véhicule facilitant l'administration du vaccin. De tels véhicules sont par exemple: la polyvinyl-pyrrolidone, ou tout adjuvant connu, c'est-à-dire toute substance permettant l'absorption plus aisée du médicament ou facilitant son action dans l'organisme.

A titre d'exemple d'autres adjuvants de ce dernier type on mentionnera encore la carboxyméthyl-cellulose, les hydroxydes et phosphates d'aluminium, ou tous autres adjuvants de ce type bien connus de l'homme de l'art. Enfin, les compositions immunogènes contiennent si besoin, un adjuvant immunologique, notamment du type muramyl peptide, ou encore l'adjuvant de Syntex® décrit par Allison et al notamment dans "Proceeding book - colloque des Cent Gardes - Octobre 1986 - PARIS - FRANCE".

L'invention vise aussi l'utilisation des molécules hybrides à titre d'antigènes dans des compositions pour le diagnostic de la présence d'anticorps résultant d'une infection par un rétrovirus IV, HTLV-I ou HTLV-II. De telles compositions peuvent être mises en oeuvre sur un échantillon constitué par exemple par un sérum de patient et l'on peut par exemple faire appel aux techniques ELISA pour effectuer la détection.

L'invention concerne aussi les séquences nucléotidiques d'ADN et d'ARN codant pour les séquences peptidiques décrites précédemment, et en particulier pour les séquences peptidiques des épitopes B préférés.

Ces séquences nucléotidiques peuvent être utilisées sous forme de sondes, ou encore peuvent entrer dans la composition de sondes pour le diagnostic d'une infection par un rétrovirus HIV.

Dans un mode de réalisation particulier du diagnostic in vitro d'une infection, par un rétrovirus on utilise les techniques d'amplification génétique, lesdites sondes étant mises en oeuvre à titre d'amorces. On peut à cet égard se reporter aux techniques décrites dans les demandes de brevet EP-A-0 200 362, EP-A-0 229 701 et EP-A-0 283 327.

L'invention vise en particulier un ADN recombinant caractérisé en ce qu'il comprend une première séquence nucléotidique codant pour un peptide immunogène issu de la glycoprotéine env et contenant un épitope B et une seconde séquence d'acides aminés codant pour un épitope T issu d'une protéine du HIV différente de la glycoprotéine env, le cas échéant sous le contrôle d'un promoteur permettant l'expression dans un hôte déterminé choisi par exemple parmi les levures, les virus, les bactéries ou les cellules eucaryotes des susdites première et seconde séquences sous la forme d'une molécule hybride.

Elle concerne également un hôte transformé par ledit ADN recombinant caractérisé en ce qu'il s'agit d'une levure, d'un virus par exemple un baculovirus, un poxvirus, un adénovirus ou un virus de l'herpès, d'une cellule eucaryote ou d'une bactérie, par exemple E.coli.

D'autres avantages et caractéristiques de l'invention apparaîtront dans les exemples qui suivent.

EXEMPLE 1: Purification de la protéine p18 recombinante de HIV-1 produite par une souche d'Escherichia coli

Un plasmide d'expression procaryote de p18 de HIV-1 (pTG2153) est construit de la façon suivante:
- le bactériophage M13TG1154 [Rautmann G. et al. AIDS Res. & Human Retroviruses 5 p117-157] porte le gène gag complet dans lequel le codon TAC codant pour le dernier acide aminé de p18 a été remplacé par un codon stop, TAG. Un site de restriction BglII est introduit immédiatement en amont du gène codant pour p18 par mutagénèse dirigée à l'aide de l'oligonucléotide suivant:

```
5' CTCGCACCCATAGATCTCCTTCTAG 3'
```

pour donner le bactériophage M13TG1161.

- un fragment BglII-PstI contenant le gène codant pour p18 est introduit dans le vecteur d'expression procaryote pTG959 dont la construction est décrite dans la publication de brevet EP-0292404, portant le promoteur PL du bactériophage Lambda, N, une séquence (ribosome binding site) synthétique, cII, lacz et ampR. Cette insertion se fait après digestion de pTG959 par BglII et PstI qui élimine la séquence cII. On obtient ainsi le plasmide pTG2153.

La souche E.coli 901 est ensuite transformée par ce plasmide d'expression procaryote de p18. Le gène de p18 se trouve alors sous le contrôle du promoteur PL régulé par son répresseur thermosensible. Après environ 2,5 heures de croissance à 30°C (jusqu'à une D.O. de 0,3 à 550 nm), l'augmentation de la température du milieu de culture à 42°C induit l'expression de la protéine p18. Après 7 heures (D.O. environ 2,4) à cette température les cellules sont récoltées par centrifugation (10 min. à 5000 tours/min). Le culot est repris dans du tampon PBS.

La protéine p18 recombinante est libérée des cellules après congélation à -80°C puis décongélation à 0-2°C ou par sonication. On effectue alors une centrifugation (20 min à 10000 tours/min) et on récupère le surnageant. On recommence une fois ces deux opérations avant de purifier la protéine.

La protéine p18 recombinante est purifiée par chromatographie d'échange de cations sur S Sepharose suivie après dilution (pour obtenir une conductivité identique à celle de PBS) d'une phase de chromatographie HPLC échangeuse de cations forts avec un rendement de près de 20%.

La protéine p18 recombinante ainsi obtenue est ensuite caractérisée par électrophorèse SDS-PAGE, HPLC en phase réserve et par détermination de sa séquence d'acides aminés en effectuant des cartes peptidiques avant et après clivage par la trypsine ou la protéase V8 de staphylococcus aureus. Ces vérifications confirment que la séquence de la protéine p18 recombinante est conforme à celle prédite par l'analyse de la séquence nucléotidique de l'ADN complémentaire utilisé. Elle est reconnue par des anticorps monoclonaux spécifiques à p18 de HIV-1 [Chassagne J. et al. J. Immunol. (1986) 186 P14442].

La protéine p18 recombinante présente un degré de pureté de 90%, elle peut être conservée dans un tampon phosphate/NaCl à -80°C et rester stable.

EXEMPLE 2: Purification de la protéine p25 recombinante de HIV-1 produite par une souche d'Escherichia coli.

Le plasmide d'expression procaryote de p25 de HIV-1 (pTG2103) est construit de la façon suivante:

- le bactériophage M13TG1124 décrit dans la demande de brevet EP-0276591 renferme un cadre de lecture qui code pour une protéine P25 allongée à son extrémité N-terminale de deux acides aminés supplémentaires: une méthionine suivie d'une glycine. Le reste de la séquence est rigoureusement identique à celle de P25 de HIV-1. On introduit un site BglII et un codon alanine à l'extrémité N-terminale du cadre de lecture de p25 (en remplacement de la glycine) par mutagénèse dirigée à l'aide de l'oligonucléotide suivant:

```
5' TAGGTGCCATAGATCTGACCTGGA 3'
```

pour obtenir le phage M13TG1126.

- un fragment BglII-EcoRI de M13TG1126 est introduit dans le plasmide pTG959 digéré par BglII et EcoRI pour donner le plasmide d'expression procaryote de P25, pTG2103.

La souche E.coli 901 est ensuite transformée par ce plasmide d'expression de P25. Le gène de P25 se trouve alors sous le contrôle du promoteur PL régulé par son répresseur thermosensible. Après environ 2,5 heures de croissance à 30°C (jusqu'à une D.O. de 0,3), l'augmentation de la température du milieu de culture à 42°C induit l'expression de la protéine P25. Après 7 heures (D.O. = 2,4) à cette température les cellules sont récoltées par centrifugation (10min à 5000 tours/min).

La protéine P25 recombinante est extraite de la fraction cytosolique après sonication ou par "French-press". Le surnageant est dialysé/diafiltré contre un tampon à faible force ionique (20mM Tris/HCl, pH8). L'adsorption de la plus grande partie des protéines de E.coli est obtenue sur un support d'échange d'anions faibles. La protéine P25 se trouve dans la fraction non adsorbée avec une pureté d'environ 80%. Cette fraction est soumise à une chromatographie sur Sepharose® chélate de métal (chargé avec Zn2 +), et équilibrée avec du tampon Tris à faible force ionique (20mM) et pH8. La protéine p25 est libérée par un gradient linéaire de glycine (0-200mM, pH8), suivi par une étape sur 500mM d'imidazole, pH8.

A la place de la chromatographie avec un chélate de métal on peut effectuer une chromatographie sur TSK orange. Cette étape peut être aussi effectuée comme étape supplémentaire de purification. La protéine est alors mise en solution dans un tampon Tris à faible force ionique, pH8. L'élution est réalisée par un gradient linéaire de NaCl (0-1M).

Les fractions riches en p25 sont mélangées, réduites à température ambiante avec 40mM de béta-mercaptoéthanol et diafiltrées contre 170mM de NaCl, 20mM de sodium phosphate, pH7. La protéine p25 recombinante ainsi purifiée présente une pureté supérieure à 90%. Elle peut être stockée à -80°C sans subir de dégradation.

La protéine p25 recombinante est caractérisée par électrophorèse sur gel (SDS-PAGE), HPLC en phase reverse, détermination de la composition en acides aminés, analyse des séquences N- et C-terminales après digestion protéolytique, focalisation isoélectrique et spectrométrie de masse.

EXEMPLE 3: Purification de la protéine nef de HIV-1 recombinante produite par une souche d'Escherichia coli.

La production de la protéine nef recombinante de HIV-1 par la souche TGE901 a été décrite dans la demande de brevet EP-0292404.

La protéine nef recombinante est extraite de la fraction cytosolique après sonication ou par "French-press". Après centrifugation on récupère le culot et le surnageant qui vont subir des traitements différents:

- le surnageant est précipité dans une solution à 35% de (NH4)2SO4 puis centrifugé à 10000 tours/min pendant 20 min. Le culot est récupéré dans du tampon HEPES-1 (HEPES, 20mM; pH8; GDP 50$\mu$M; Mg2 + 5mM; DTT 10mM; EDTA 100 $\mu$M; EGTA 100 $\mu$M; NaN3 100 $\mu$M et PMSF 100 $\mu$M),

Le culot est solubilisé pendant 12 à 16 heures à température ambiante dans une solution d'urée 8M puis est soumis pendant 24 heures à une dialyse à 4°C dans la solution HEPES-1. Après centrifugation à 10000 tours/min pendant 20 min on récupère le surnageant.

Les surnageants obtenus dans ces deux étapes sont par la suite traités de façon identique.

On effectue tout d'abord une chromatographie échangeuse d'anions sur DEAE Spherodex® équilibré avec HEPES-1. La protéine nef est éluée par gradient de NaCl (0 à 1M) puis les fractions riches en protéine nef sont soumises à une chromatographie d'affinité sur Cibacron Blue F3GA Agarose équilibré avec le tampon HEPES-1 sans DTT, EDTA et EGTA. La protéine nef est ensuite éluée par gradient de NaCl (0 à 1,5M) dans le tampon HEPES-1 puis les fractions riches en protéine nef sont soumises à une chromatographie d'affinité sur chelate de métal (Zn2 +) en utilisant le tampon HEPES-2 (pH7, HEPES 20mM, NaCl 0,5M, imidazole 1 mM, GDP 50 $\mu$M et Mg2 + 100 $\mu$M).

La protéine nef est éluée par gradient d'imidazole (1 à 20mM) et les fractions riches sont diafiltrées. Elle est ensuite reprise dans du tampon HEPES 20mM (pH8, GDP 50 $\mu$M, Mg2 + 100 $\mu$M et DTT 5mM).

La protéine nef recombinante ainsi isolée est analysée par Western blot. Sa pureté, déterminée par HPLC phase réverse est supérieure à 95% et par SDS PAGE de 90%. La séquence N-terminale ainsi que l'activité de liaison avec GTP sont aussi vérifiées et trouvées conformes.

EXEMPLE 4: Couplage covalent de peptides de HIV issus de la région de la glycoprotéine formant une boucle de Putney, avec P18gag ou avec P27nef.

2mg de P18 ou de P27nef dans 4ml de tampon 0.1M phosphate 0.1M NaCl pH 7,5 sont traités par addition de 20$\mu$l d'une solution de SPDP à 10mg/ml dans l'éthanol. Après 30 min. à 25°C le mélange est passé sur Sephadex® G-25. Les fractions exclues sont reprises et mélangées avec le cocktail des 21 peptides HIV correspondant aux 21 séquences connues de l'épitope de Putney chacun comportant une cystéine de part et d'autre de cette séquence et présents à raison de 100$\mu$g chacun. Ledit cocktail a été au préalable réduit par traitement au $\beta$-mercaptoéthanol O-1M bicarbonate d'ammonium puis rapidement lyophilisé.

Le mélange P18-peptides ou P27-peptides est incubé la nuit à 25° puis passé sur Sephadex® G-50 après centrifugation du précipité insoluble qui s'est formé pendant la nuit. Les fractions exclues sont reprises, ainsi que le précipité pour être injectées au lapin à raison de 200$\mu$g/dose par voie sous cutanée en présence d'adjuvant de Freund (complet puis incomplet). Les fractions injectées ont été testées par RIA pour détecter la présence des peptides comme décrit à l'exemple suivant:

EXEMPLE 5: Couplage covalent de peptides HIV-1 à la Serum Albumine Bovine (BSA).

1mg de BSA (500 µG/ml dans 0.1M phosphate-0,1 NaCl à pH 7,5) a été mélangé avec du N-succinimidyl-3-(2 pyridyldithio)-propionate (SPDP; 10%l de 10 mg/ml de solution dans l'éthanol) pendant 30 minutes à 25°C. Les dérivés de BSA ont été séparés de l'excès de SPDP par chromatographie d'exclusion sur Sephadex G25. Les fractions correspondant au volume exclu de la colonne, ont été mélangées avec 1mg des peptides ayant des résidus cystéine et réduits avec le 2 mercaptoéthanol (10mg/ml) dans 0,1M de bicarbonate d'ammonium. On a ensuite procédé à une lyophilisation rapide, avant l'utilisation. Le mélange de BSA traitée au SPDP et de peptides réduits a été conservé pendant la nuit à 25°C, puis chromatographié sur une colonne de Sephadex® G50. Les fractions retenues correspondant au volume exclu de la colonne ont été testées pour détecter la présence des peptides. A cet effet, des billes de polystyrène ont été recouvertes avec les fractions récupérées diluées 10 fois en série dans 0,1M Tris, à pH 8,8 pendant une nuit à 20°C. Les billes ont ensuite été recouvertes avec BSA (10mg/ml) et de la gélatine (2,5mg/ml). Les peptides fixés aux billes ont été détectés par RIA.

EXEMPLE 6: Epitope de p27 nef reconnu par les lymphocytes T auxiliaires ("helper").

La spécificité particulière de la réponse proliférative des lymphocytes à la protéine nef p27 a été recherchée. Une série de 14 peptides synthétiques se chevauchant, couvrant la totalité de la séquence du produit du gène nef de HIV-1 LAV-BRU a été testée pour détecter leur capacité à induire des réponses prolifératives des lymphocytes du sang périfirique PBL (Peripheral Blood Lymphocytes) chez deux chimpanzés à différents moments après l'injection d'antigène nef.

Ces chimpanzés ont reçu la P27 nef purifiée, mélangée avec l'adjuvant Syntex® à base de MDP-Thréonyl. 3 injections ont été pratiquées à 1 mois d'intervalle, suivies d'un rappel à 6 mois.

La concentration en peptides nécessaire pour observer une réponse optimale a été tout d'abord déterminée comme étant environ 50µg/ml, en utilisant des peptides dont on connaissait la capacité à induire une réponse positive ou négative.

Dans des tests indépendants, les lymphocytes du chimpanzé 479 ont montré qu'ils réagissaient fortement avec les peptides PF16 (résidus 171-205), PF17 (141-205) et PF63 (185-205). Une prolifération plus faible (inférieure à 25 à 50% RR, Réponse Relative) a été notée avec deux des trois échantillons de PBL vis-à-vis des peptides PF15 (118-167) et avec trois des quatre échantillons vis-à-vis des peptides PF18 (93-122). Aucune prolifération significative n'a été détectée de façon reproductible avec les autres peptides utilisés (tableau 1).

Des résultats comparables ont été obtenus avec les PBL du chimpanzé 433.

On a ainsi vérifié qu'aucun des peptides stimulants n'était mitogénique non spécifique pour les cellules T à la concentration utilisée: les PBL du chimpanzé contrôle (411) qui a été immunisé uniquement contre le virus de vaccine, et qui a reçu des injections répétées d'adjuvant n'ont pas proliféré en réponse à ces peptides.

Ces données montrent la reconnaissance par les cellules T auxiliaires des 2 chimpanzés, d'au moins 2 épitopes présents sur les cellules T de la protéine p27 nef: 1 épitope semble localisé à l'intérieur de la séquence de 60 acides aminés C-terminale de la molécule et plus probablement au niveau des 20 derniers acides aminés comme le montre l'antigénicité de PF63. L'autre épitope pourrait être localisé aux environs d'une région recouvant à la fois PF18 et PF15 (109-122). Ces peptides sont avec avantage combinés avec des épitopes B tels qu'ils ont été définis plus haut.

14

# EP 0 439 601 B1

## TABLEAU 1

<u>REPONSES PROLIFERATIVES DES PBL DES CHIMPANZES n° 479 à P27nef (500µg/ml) ET AUX PEPTIDES SYNTHETIQUES (50µg/ml) DE CETTE PROTEINE</u>

<u>REPONSE RELATIVE A P27(%) A:</u>

| Peptide | position a.a | semaine 4 | semaine 12 | semaine 14(1) | semaine 41 |
|---------|-------------|-----------|------------|---------------|------------|
| PF 12 | 1-66 | 37 | 12 | - | 21 |
| PF 11 | 1-31 | 0 | 16 | - | 12 |
| PF 3 | 1-17 | (2) | 0 | - | 3 |
| PF 5 | 17-35 | - | 2 | - | 0 |
| PF 13 | 32-64 | 8 | 17 | - | 11 |
| PF 6 | 35-52 | - | 0 | - | 5 |
| PF 14 | 65-109 | 13 | 28 | - | 13 |
| PF 8 | 88-105 | - | 0 | - | 0 |
| PF 18 | 93-122 | 29 | 42 | 51 | 20 |
| PF 15 | 118-167 | 28 | 40 | - | 25 |
| PF 17 | 141-205 | 71 | 155 | 222 | 81 |
| PF 62 | 147-172 | - | 5 | 0 | 3 |
| PF 16 | 171-205 | 98 | 174 | 59 | 52 |
| PF 63 | 185-205 | - | 78 | 125 | 54 |
| réacti-vité de PBL (3) | contrôle | 3.8 | 2.1 | 5.0 | 2.9 |
| | P27 | 40.9 | 17.5 | 23.7 | 23.2 |

(1) % RR sur cette colonne est dérivé des réponses prolifératives à 50µg/ml de peptides.
(2) non déterminé
(3) incorporation de [$^3$H] thymidine (cpmx10$^{-3}$).

## Revendications

1. Molécule hybride caractérisée en ce qu'elle comprend au moins un peptide immunogène contenant un épitope B issu d'une glycoprotéine d'enveloppe d'un rétrovirus HIV, SIV pathogène, HTLV-I ou HTLV-II, et au moins un épitope T issu d'une protéine, autre qu'une glycoprotéine d'enveloppe, d'un rétrovirus HIV, SIV pathogène, HTLV-I ou HTLV-II, les deux protéines étant ou non issues du même rétrovirus.

2. Molécule hybride selon la revendication 1, caractérisée en ce que l'épitope T est issu d'une proteine choisie parmi les protéines codées par les gènes <u>nef</u>, <u>gag</u>, <u>tat</u>, <u>rev</u>, <u>vif</u>, <u>pol</u>, <u>vpr</u>, <u>vpu</u>, <u>vpx</u> de HIV, SIV, HTLV-I ou HTLV-II.

3. Molécule hybride selon la revendication 1 ou 2 caractérisée en ce que le peptide immunogène et l'épitope T sont couplés chimiquement.

4. Molécule hybride selon l'une quelconque des revendications 1 à 3, caractérisée en ce que le peptide immunogène contenant l'épitope B correspond à un épitope majeur de neutralisation d'une glycoprotéine d'enveloppe.

**5.** Molécule hybride selon la revendication 4, caractérisée en ce que le peptide immunogène contenant l'épitope B comprend une partie d'un épitope majeur de neutralisation d'une glycoprotéine d'enveloppe dès lors que cette partie est suffisante pour induire _in vivo_ la production d'anticorps aptes à neutraliser le rétrovirus correspondant.

**6.** Molécule hybride selon l'une quelconque des revendications 1 à 5, caractérisée en ce que le peptide contenant l'épitope B comprend l'une des séquences suivantes ou une partie de ces séquences comprenant l'épitope B :

```
NTRKR  IRIQRGPGRA  FVTIGK-IGN
NTRKS  IRIQRGPGRA  FVTIGK-IGN
NTRKK  IRIQRGPGRA  FVTIGK-IGN
NTRGS  IRIQRGPGRA  FVTIGK-IGN
NTRKS  IYI--GPGRA  FHTTGRIIGD
NVRRS  LSI--GPGRA  FRTRE-IIGI
NTRRG  IHF--GPGQA  LYTTGIV-GD
NTRQR  TPI--GLGQS  LYTTRSR-SI
NTRKS  ITK--GPGRV  IYATGQIIGD
NTRKR  ITM--GPGRV  YYTTGQIIGD
DKRQS  TPI--GLGQA  LYTTRGRTKI
DKKIR  QSIRIGPGKV  FYAKGG---I
NTKKG  IAI--GPGRT  LYAREKIIGD
HTRKR  VTL--GPGRV  WYTTGEILGN
NTRRG  SHF--GPGQA  LYTTGIVGDI
KITSRQTPI--GLGQA  LYTTRIKGDI
NVRRR  HIHI-GPGRA  FYTGEIRNI
NTRQS  TPI--GLGQA  LYTTRTKSI
NTTRS  IHI--GPGRA  FYATGDIIGTI
NKRKR  IHI--GPGRA  FYTTKNIIGDI
```

**7.** Molécule hybride selon l'une quelconque des revendications 1 à 5, caractérisée en ce que le peptide contenant l'épitope B comprend l'une des séquences suivantes ou une partie de ces séquences comprenant l'épitope B :

```
TRPNNNTRKR  IRIQRGPGRA  FVTIGK-IGN  M-RQAH
TRPNNNTRKS  IRIQRGPGRA  FVTIGK-IGN  M-RQAH
TRPNNNTRKK  IRIQRGPGRA  FVTIGK-IGN  M-RQAH
TRPNNNTRGS  IRIQRGPGRA  FVTIGK-IGN  M-RQAH
TRPNNNTRKS  IYI--GPGRA  FHTTGRIIGD  -IRKAH
TRPYNNVRRS  LSI--GPGRA  FRTRE-IIGI  -IRQAH
TRPGNNTRRG  IHF--GPGQA  LYTTGIV-GD  -IRRAY
ARPYQNTRQR  TPI--GLGQS  LYTTRSR-SI  -IGQAH
TRPNNNTRKS  ITK--GPGRV  IYATGQIIGD  -IRKAH
TRPNNNTRKR  ITM--GPGRV  YYTTGQIIGD  -IRPAH
TRPGSDKRQS  TPI--GLGQA  LYTTRGRTKI  -IGQAH
TRPGSDKKIR  QSIRIGPGKV  FYAKGG---I  -TGQAH
TRPNNNTKKG  IAI--GPGRT  LYAREKIIGD  -IRQAH

TRPNNHTRKR  VTL--GPGRV  WYTTGEILGN  -IRQAH
TRPGNNTRRG_SHF--GPGQA  LYTTGIVGDI  -RRAY
TRPDNKITSRQ-TPI-GLGQA  LYTTRIKGDI  -RQAY
TRPNNNVRRR-HIHI-GPGRA  FYTGEIRNI   -RQAH
TRPYKNTRQS-TPI--GLGQA  LYTTRTKSI   -GQAH
TRPNNNTTRS-IHI--GPGRA  FYATGDIIGTIRQAH
TRPNYNKRKR-IHI--GPGRA  FYTTKNIIGDIRQAH
```

8. Molécule hybride selon l'une quelconque des revendications 1 à 7, caractérisée en ce qu'elle comprend les régions peptidiques de la glycoprotéine d'enveloppe de HIV-1 comprenant respectivement les acides aminés 267 et 128 participant à la formation de l'épitope majeur de neutralisation.

9. Molécule hybride selon l'une quelconque des revendications 1 à 5, caractérisée en ce qu'elle comprend un épitope mineur de neutralisation B, en particulier un épitope B issu d'une région conservée d'une glycoprotéine d'enveloppe.

10. Molécule hybride selon la revendication 9, caractérisée en ce que l'épitope mineur comprend l'une des séquences suivantes :

```
YDRPEGIEEEGGERDRDRSG

VAPTKAKRRVVQREKRAVGIGALFLGFLGAG

CTHGIRPVVSTQLLLNGSLAE

STQLLLNGSLAEEEVVIRC
```

**11.** Molécule hybride selon l'une quelconque des revendications 1 à 10, caractérisée en ce que l'épitope T est en totalité ou en partie constitué par une protéine spécifique de HIV ou de SIV, notamment choisie parmi les protéines codées par les gènes gag, tat, rev, vif, pol, vpr, vpu, vpx.

**12.** Molécule hybride selon l'une quelconque des revendications 1 à 10, caractérisée en ce que l'épitope T, est en totalité ou en partie constitué par la protéine codée par le gène nef ou d'un antigène dérivé.

**13.** Molécule hybride selon l'une quelconque des revendications 1 à 10, caractérisée en ce que l'épitope T est en totalité ou en partie constitué par une protéine gag, choisie notamment parmi P55, P25, P18 ou P12 de HIV-1, et les protéines codées par les gènes correspondants de HIV-2 ou de SIV.

**14.** Molécule hybride selon l'une quelconque des revendications 1 à 13, caractérisée en ce qu'elle comporte en outre une séquence ou molécule porteuse autorisant l'accroissement du pouvoir immunogène de l'ensemble et à laquelle sont couplés le peptide immunogène et l'épitope T ou à laquelle l'un ou l'autre, voire les deux à la fois, sont incorporés.

**15.** Molécule hybride selon la revendication 14, caractérisée en ce que la molécule porteuse est constituée par une partie ou la totalité de la séquence d'acides animés de la protéine à laquelle l'épitope T appartient naturellement dans le rétrovirus HIV ou SIV natif dont elle provient.

**16.** Molécule hybride selon la revendication 15, caractérisée en ce que la séquence d'acides aminés est celle de la protéine nef d'un HIV OU SIV.

**17.** Molécule hybride selon la revendication 15, caractérisée en ce que la séquence d'acides aminés est celle d'une protéine gag d'un HIV ou SIV.

**18.** Molécule hybride selon l'une quelconque des revendications 1 à 11, caractérisée en ce que le peptide portant l'épitope B et l'épitope T sont greffés par couplage chimique sur une molécule porteuse, telle que l'anatoxine tétanique, les antigènes HBs, ou HBc, l'hémocyanine ou l'albumine humaine.

**19.** Composition immunogène comprenant une pluralité de molécules hybrides conforme à l'une quelconque des revendications 1 à 18.

**20.** Composition selon la revendication 19, caractérisée en ce qu'elle contient la totalité des molécules hybrides définies dans l'une quelconque des revendications 6 à 9.

**21.** Composition selon la revendication 19, caractérisée en ce qu'elle contient en outre au moins l'une des molécules hybrides définies dans la revendication 8 ou la revendication 9.

**22.** ADN recombinant caractérisé en ce qu'il comprend une première séquence nucléotidique codant pour un peptide immunogène issu d'une glycoprotéine d'enveloppe d'un rétrovirus HIV, SIV pathogène, HTLV-I ou HTLV-II et contenant un épitope B et une seconde séquence d'acides aminés codant pour un épitope T issu d'une protéine, autre qu'une glycoprotéine d'enveloppe, d'un rétrovirus HIV, SIV pathogène, HTLV-I ou HTLV-II, le cas échéant sous le contrôle d'un promoteur permettant l'expression des susdites première et seconde séquences sous la forme d'une molécule hybride conforme à l'une des molécules selon l'une quelconque des revendications 1 à 20, dans un hôte déterminé choisi par exemple parmi les levures, les virus, les bactéries ou les cellules eucaryotes.

**23.** Hôte transformé par un ADN recombinant selon la revendication 22, caractérisé en ce qu'il s'agit d'une levure, d'un virus, par exemple un baculovirus, un poxvirus, un adénovirus ou un herpès virus, d'une cellule eucaryote ou d'une bactérie, par exemple E. coli.

**24.** Composition immunogène capable d'induire in vivo la production d'anticorps neutralisants et protecteurs vis-à-vis d'un rétrovirus HIV, SIV pathogène, HTLV-I, ou HTLV-II, en particulier d'un rétrovirus HIV pathogène pour l'homme, caractérisée en ce qu'elle comprend en tant que principe actif une molécule hybride selon l'une quelconque des revendications 1 à 7, ou un ensemble de molécules hybrides selon l'une quelconque des revendications 19 à 21, en association avec un véhicule pharmaceutique acceptable.

**25.** Séquences nucléotidiques d'ADN ou d'ARN, codant pour l'une des séquences peptidiques selon l'une quelconque des revendications 1 à 10 ou l'une des séquences nucléotidiques codant pour les épitopes B selon l'une quelconque des revendications 6, 7, 9 ou 10.

**26.** Composition pour le diagnostic in vitro d'une infection par un rétrovirus HIV, caractérisée en ce qu'elle comprend une ou plusieurs séquences nucléotidiques selon la revendication 25 ou une molécule hybride selon l'une quelconque des revendications 1 à 18.

**Claims**

**1.** Hybrid molecule characterized in that it comprises at least one immunogenic peptide containing a B epitope derived from a envelope glycoprotein of a HIV, pathogenic SIV, HTLV-I or HTLV-II retrovirus, and at least one T epitope derived from a protein other than a envelope glycoprotein of a HIV, pathogenic SIV, HTLV-I or HTLV-II retrovirus, the two proteins being derived from the same or different retroviruses.

**2.** Hybrid molecule according to Claim 1, characterized in that the T epitope is derived from a protein selected from the proteins encoded in the nef, gag, tat, rev, vif, pol, vpr, vpu, vpx genes of HIV, SIV, HTLV-I or HTLV-II.

**3.** Hybrid molecule according to Claim 1 or 2 characterized in that the immunogenic peptide and the T epitope are chemically coupled.

**4.** Hybrid molecule according to any one of the Claims 1 to 3, characterized in that the immunogenic peptide containing the B epitope corresponds to a major neutralizing epitope of a envelope glycoprotein.

**5.** Hybrid molecule according to Claim 4, characterized in that the immunogenic peptide containing the B epitope comprises a part of the major neutralizing epitope of a envelope glycoprotein provided that this part is sufficient to induce in vivo the production of antibodies capable of neutralizing the corresponding retrovirus.

**6.** Hybrid molecule according to any one of the Claims 1 to 5, characterized in that the peptide containing the B epitope comprises one of the following sequences or a part of these sequences containing the B epitope:

```
NTRKR  IRIQRGPGRA  FVTIGK-IGN

NTRKS  IRIQRGPGRA  FVTIGK-IGN

NTRKK  IRIQRGPGRA  FVTIGK-IGN

NTRGS  IRIQRGPGRA  FVTIGK-IGN

NTRKS  IYI--GPGRA  FHTTGRIIGD

NVRRS  LSI--GPGRA  FRTRE-IIGI

NTRRG  IHF--GPGQA  LYTTGIV-GD

NTRQR  TPI--GLGQS  LYTTRSR-SI

NTRKS  ITK--GPGRV  IYATGQIIGD

NTRKR  ITM--GPGRV  YYTTGQIIGD
```

```
DKRQS TPI--GLGQA LYTTRGRTKI
DKKIR QSIKIGPGKV FYAKGG---I
NTKKG IAI--GPGRT LYAREKIIGD
HTRKR VTL--GPGRV WYTTGEILGN
NTRRG SHF--GPGQA LYTTGIVGDI
KITSRQTPI--GLGQA LYTTRIKGDI
NVRRR HIHI-GPGRA FYTGEIRNI
NTRQS TPI--GLGQA LYTTRTKSI
NTTRS IHI--GPGRA FYATGDIIGTI
NKRKR IHI--GPGRA FYTTKNIIGDI
```

7. Hybrid molecule according to any one of the Claims 1 to 5, characterized in that the peptide containing the B epitope includes one of the following sequences or a part of these sequences containing the B epitope:

```
TRPNNNTRKR IRIQRGPGRA FVTIGK-IGN M-RQAH
TRPNNNTRKS IRIQRGPGRA FVTIGK-IGN M-RQAH
TRPNNNTRKK IRIQRGPGRA FVTIGK-IGN M-RQAH
TRPNNNTRGS IRIQRGPGRA FVTIGK-IGN M-RQAH
TRPNNNTRKS IYI--GPGRA FHTTGRIIGD -IRKAH
TRPYNNVRRS LSI--GPGRA FRTRE-IIGI -IRQAH
TRPGNNTRRG IHF--GPGQA LYTTGIV-GD -IRRAY
ARPYQNTRQR TPI--GLGQS LYTTRSR-SI -IGQAH
TRPNNNTRKS ITK--GPGRV IYATGQIIGD -IRKAH
TRPNNNTRKR ITM--GPGRV YYTTGQIIGD -IRPAH
TRPGSDKRQS TPI--GLGQA LYTTRGRTKI -IGQAH
TRPGSDKKIR QSIRIGPGKV FYAKGG---I -TGQAH
TRPNNNTKKG IAI--GPGRT LYAREKIIGD -IRQAH
TRPNNHTRKR VTL--GPGRV WYTTGEILGN -IRQAH
TRPGNNTRRG_SHF--GPGQA LYTTGIVGDI -RRAY
TRPDNKITSRQ-TPI-GLGQA LYTTRIKGDI -RQAY
```

```
TRPNNNVRRR-HIHI-GPGRA  FYTGEIRNI  -RQAH
TRPYKNTRQS-TPI--GLGQA  LYTTRTKSI  -GQAH
TRPNNNTTRS-IHI--GPGRA  FYATGDIIGTIRQAH
TRPNYNKRKR-IHI--GPGRA  FYTTKNIIGDIRQAH
```

8.  Hybrid molecule according to any one of the Claims 1 to 7, characterized in that it comprises the peptide regions of the envelope glycopeptide of HIV-1 including the amino acids 267 and 128, respectively, which participate in the formation of the major neutralizing epitope.

9.  Hybrid molecule according to any one of the Claims 1 to 5, characterized in that it comprises a minor neutralizing B epitope, in particular a B epitope derived from a conserved region of an envelope glycoprotein.

10. Hybrid molecule according to Claim 9, characterized in that the minor epitope includes one of the following sequences:

```
YDRPEGIEEEGGERDRDRSG

VAPTKAKRRVVQREKRAVGIGALFLGFLGAG

CTHGIRPVVSTQLLLNGSLAE

STQLLLNGSLAEEEVVIRC
```

11. Hybrid molecule according to any one of the Claims 1 to 10, characterized in that the T epitope is constituted in whole or in part by a specific protein of HIV or SIV, selected in particular from the proteins encoded in the gag, tat, rev, vif, pol, vpr, vpu, vpx genes.

12. Hybrid molecule according to any one of the Claims 1 to 10, characterized in that the T epitope is constituted in whole or in part by the protein encoded in the nef gene or a derived antigen.

13. Hybrid molecule according to any one of the Claims 1 to 10, characterized in that the T epitope is constituted in whole or in part by a gag, protein, selected in particular from p55, p25, p18 or p12 of HIV-1, and the proteins encoded in the corresponding genes of HIV-2 or SIV.

14. Hybrid molecule according to any one of the Claims 1 to 13, characterized in that it contains, in addition, a sequence or carrier molecule which enhances the immunogenic potency of the ensemble and to which are coupled the immunogenic peptide and the T epitope or in which one or the other or both are incorporated.

15. Hybrid molecule according to Claim 14, characterized in that the carrier molecule is constituted by a part or all of the amino acid sequence of the protein to which the T epitope naturally belongs in the native HIV or SIV retrovirus from which it is derived.

16. Hybrid molecule according to Claim 15, characterized in that the amino acid sequence is that of the nef gene of a HIV or a SIV.

17. Hybrid molecule according to Claim 15, characterized in that the amino acid sequence is that of a gag protein of a HIV or a SIV.

18. Hybrid molecule according to any one of the Claims 1 to 11, characterized in that the peptide bearing the B epitope and the T epitope are grafted on to a carrier molecule, such as tetanus toxoid, the HBs or HBc antigens, hemocyanin or human albumin, by chemical coupling.

19. Immunogenic composition containing several hybrid molecules conforming to any one of the Claims 1 to 18.

20. Composition according to Claim 19, characterized in that it contains all of the hybrid molecules defined in any one of the Claims 6 to 9.

21. Composition according to Claim 19, characterized in that it contains, in addition, at least one of the hybrid molecules defined in Claim 8 or Claim 9.

22. Recombinant DNA characterized in that it comprises a first nucleotide sequence coding for an immunogenic peptide derived from an envelope glycoprotein of a HIV, pathogenic SIV, HTLV-I or HTLV-II retrovirus and containing a B epitope and a second sequence nucleotide sequence coding for a T epitope derived from a protein other than an envelope glycoprotein of a HIV, pathogenic SIV, HTLV-I or HTLV-II retrovirus, optionally under the control of a promoter enabling the above-mentioned first and second sequences to be expressed in the form of a hybrid molecule in conformity with one of the molecules according to any one of the Claims 1 to 20, in a defined host selected for example from the yeasts, the viruses, the bacteria or eukaryotic cells.

23. Host transformed by a recombinant DNA according to Claim 22, characterized in that it is a yeast, a virus for example a baculovirus, a pox virus, an adenovirus or a herpès virus, a eukaryotic cell or a bacterium, for example E. coli.

24. Immunogenic composition capable of inducing in vivo the production of neutralizing and protective antibodies against a HIV, pathogenic SIV, HTLV-I or HTLV-II retrovirus, in particular a HIV retrovirus pathogenic for man, characterized in that it contains as active ingredient a hybrid molecule according to any one of the Claims 1 to 7, or a set of hybrid molecules according to any one of the Claims 19 to 21, in combination with an acceptable pharmaceutical vehicle.

25. DNA or RNA nucleotide sequences coding for one of the peptide sequences according to any one of the Claims 1 to 10 or one of the nucleotide sequences coding for the B epitopes according to any one of the Claims 6, 7, 9 or 10.

26. Composition for the in vitro diagnosis of an infection by a HIV retrovirus, characterized in that it contains one or more nucleotides sequences according to the Claim 25 or a hybrid molecule according to any one of the Claims 1 to 18.

**Patentansprüche**

1. Hybridmolekül, dadurch gekennzeichnet, daß es mindestens ein immunogenes Peptid umfaßt, welches ein Epitop B enthält, das von einem Hüllglykoprotein eines der Retroviren HIV, pathogenes SIV, HTLV-I oder HTLV-II stammt, und mindestens ein Epitop T, das von einem anderen Protein als einem Hüllglykoprotein eines der Retroviren HIV, pathogenes SIV, HTLV-I oder HTLV-2 stammt, wobei die beiden Proteine nicht vom gleichen Retrovirus stammen.

2. Hybridmolekül nach Anspruch 1, dadurch gekennzeichnet, daß das Epitop T von einem Protein stammt, das ausgewählt ist aus den Proteinen, die von den Genen nef, gag, tat, rev, vif, pol, vpr, vpu, vpx von HIV, SIV, HTLV-I, oder HTLV-2 codiert werden.

3. Hybridmolekül nach Anspruch 1 oder 2, dadurch gekennzeichnet, daß das immunogene Peptid und das Epitop T chemisch verknüpft sind.

4. Hybridmolekül nach einem der Ansprüche 1 bis 3, dadurch gekennzeichnet, daß das das Epitop B enthaltende immunogene Peptid einem Hauptepitop für die Neutralisation eines Hüllglykoproteins entspricht.

**5.** Hybridmolekül nach Anspruch 4, dadurch gekennzeichnet, daß das das Epitop B enthaltende immunogene Peptid einen Teil eines Hauptepitops für die Neutralisation eines Hüllglykoproteins umfaßt, wobei dieser Teil ausreicht, um in vivo die Produktion von Antikörpern zu induzieren, die das entsprechende Retrovirus neutralisieren können.

**6.** Hybridmolekül nach einem der Ansprüche 1 bis 5, dadurch gekennzeichnet, daß das das Epitop B enthaltende Peptid eine der folgenden Sequenzen oder einen das Epitop B umfassenden Teil dieser Sequenzen enthält:

```
NTRKR IRIQRGPGRA FVTIGK-IGN
NTRKS IRIQRGPGRA FVTIGK-IGN
NTRKK IRIQRGPGRA FVTIGK-IGN
NTRGS IRIQRGPGRA FVTIGK-IGN
NTRKS IYI--GPGRA FHTTGRIIGD
NVRRS LSI--GPGRA FRTRE-IIGI
NTRRG IHF--GPGQA LYTTGIV-GD
NTRQR TPI--GLGQS LYTTRSR-SI
NTRKS ITK--GPGRV IYATGQIIGD
NTRKR ITM--GPGRV YYTTGQIIGD
DKRQS TPI--GLGQA LYTTRGRTKI
DKKIR QSIKIGPGKV FYAKGG---I
NTKKG IAI--GPGRT LYAREKIIGD
HTRKR VTL--GPGRV WYTTGEILGN
NTRRG SHF--GPGQA LYTTGIVGDI
KITSRQTPI--GLGQA LYTTRIKGDI
NVRRR HIHI-GPGRA FYTGEIRNI
NTRQS TPI--GLGQA LYTTRTKSI
NTTRS IHI--GPGRA FYATGDIIGTI
NKRKR IHI--GPGRA FYTTKNIIGDI
```

**7.** Hybridmolekül nach einem der Ansprüche 1 bis 5, dadurch gekennzeichnet, daß das das Epitop B enthaltende Peptid eine der folgenden Sequenzen oder einen das Epitop B umfassenden Teil dieser Sequenzen enthält:

```
TRPNNNTRKR  IRIQRGPGRA  FVTIGK-IGN  M-RQAH

TRPNNNTRKS  IRIQRGPGRA  FVTIGK-IGN  M-RQAH

TRPNNNTRKK  IRIQRGPGRA  FVTIGK-IGN  M-RQAH

TRPNNNTRGS  IRIQRGPGRA  FVTIGK-IGN  M-RQAH

TRPNNNTRKS  IYI--GPGRA  FHTTGRIIGD  -IRKAH

TRPYNNVRRS  LSI--GPGRA  FRTRE-IIGI  -IRQAE

TRPGNNTRRG  IHF--GPGQA  LYTTGIV-GD  -IRRAY

ARPYQNTRQR  TPI--GLGQS  LYTTRSR-SI  -IGQAH

TRPNNNTRKS  ITK--GPGRV  IYATGQIIGD  -IRKAH

TRPNNNTRKR  ITM--GPGRV  YYTTGQIIGD  -IRPAH

TRPGSDKRQS  TPI--GLGQA  LYTTRGRTKI  -IGQAH

TRPGSDKKIR  QSIRIGPGKV  FYAKGG---I  -TGQAH

TRPNNNTKRG  IAI--GPGRT  LYAREKIIGD  -IRQAH

TRPNNHTRKR  VTL--GPGRV  WYTTGEILGN  -IRQAH

TRPGNNTRRG_SHF--GPGQA  LYTTGIVGDI  -RRAY

TRPDNKITSRQ-TPI-GLGQA  LYTTRIKGDI  -RQAY

TRPNNNVRRA_HIHI-GPGRA  FYTGEIRNI   -RQAH

TRPYKNTRQS-TPI--GLGQA  LYTTRTKSI   -GQAH

TRPNNNTTRS-IHI--GPGRA  FYATGDIIGTIRQAH

TRPNYNKRKR-IHI--GPGRA  FYTTKNIIGDIRQAH
```

**8.** Hybridmolekül nach einem der Ansprüche 1 bis 7, dadurch gekennzeichnet, daß es die Peptidbereiche des Hüllglykoproteins von HIV-1 umfaßt, die die Aminosäure 267 und 128 enthalten, die an der Bildung des Hauptepitops für die Neutralisation beteiligt sind.

**9.** Hybridmolekül nach einem der Ansprüche 1 bis 5, dadurch gekennzeichnet, daß es ein Nebenepitop B für die Neutralisation umfaßt, insbesondere ein Epitop B, das von einem konservierten Bereich eines Hüllglykoproteins stammt.

**10.** Hybridmolekül nach Anspruch 9, dadurch gekennzeichnet, daß das Nebenepitop eine der folgenden Sequenzen umfaßt:

EP 0 439 601 B1

YDRPEGIEEEGGERDRDRSG

VAPTKAKRRVVQREKRAVGIGALFLGFLGAG

CTHGIRPVVSTQLLLNGSLAE

STQLLLNGSLAEEEVVIRC

11. Hybridmolekül nach einem der Ansprüche 1 bis 10, dadurch gekennzeichnet, daß das Epitop T insgesamt oder zum Teil aus einem spezifischen HIV- oder SIV-Protein besteht, insbesondere ausgewählt aus den Proteinen, die von den Genen gag, tat, rev, vif, pol, vpr, vpu, vpx codiert werden.

12. Hybridmolekül nach einem der Ansprüche 1 bis 10, dadurch gekennzeichnet, daß das Epitop T insgesamt oder zum Teil aus dem Protein besteht, das von dem Gen nef codiert wird, oder einem abgeleiteten Antigen.

13. Hybridmolekül nach einem der Ansprüche 1 bis 10, dadurch gekennzeichnet, daß das Epitop T insgesamt oder zum Teil aus einem Protein gag besteht, ausgewählt insbesondere aus P55, P25, P18 oder P12 von HIV-1, und aus den Proteinen, die von den entsprechenden HIV-2- oder SIV-Genen codiert werden.

14. Hybridmolekül nach einem der Ansprüche 1 bis 13, dadurch gekennzeichnet, daß es außerdem eine Sequenz oder ein Trägermolekül umfaßt, das die Erhöhung der immunogenen Wirkung des Gemisches ermöglicht und an das das immunogene Peptid und das Epitop T geknüpft sind oder in das das eine oder das andere oder beide eingebaut sind.

15. Hybridmolekül nach Anspruch 14, dadurch gekennzeichnet, daß das Trägermolekül aus einem Teil oder der Gesamtheit der Aminosäuresequenz des Proteins besteht, zu dem das Epitop T natürlicherweise im nativen Retrovirus HIV oder SIV gehört, von dem es abstammt.

16. Hybridmolekül nach Anspruch 15, dadurch gekennzeichnet, daß die Aminosäuresequenz die des Proteins nef vom HIV oder SIV ist.

17. Hybridmolekül nach Anspruch 15, dadurch gekennzeichnet, daß die Aminosäuresequenz die eines Proteins gag von HIV oder SIV ist.

18. Hybridmolekül nach einem der Ansprüche 1 bis 11, dadurch gekennzeichnet, daß das das Epitop B oder das Epitop T tragende Peptid durch chemische Verknüpfung auf ein Trägermolekül aufgebracht wird, wie das Tetanusanatoxin, die Antigene HBs oder HBc, Hemocyanin oder menschliches Albumin.

19. Immunogenes Mittel, umfassend eine Vielzahl von Hybridmolekülen nach einem der Ansprüche 1 bis 18.

20. Mittel nach Anspruch 19, dadurch gekennzeichnet, daß es sämtliche Hybridmoleküle gemäß der Definition in einem der Ansprüche 6 bis 9 enthält.

21. Mittel nach Anspruch 19, dadurch gekennzeichnet, daß es außerdem mindestens ein Hybridmolekül gemäß der Definition in einem der Ansprüche 8 oder 9 enthält.

22. Rekombinante DNA, dadurch gekennzeichnet, daß sie eine erste Nucleotidsequenz umfaßt, die ein immunogenes Peptid codiert, welches von einem Hüllglykoprotein der Retroviren HIV, pathogenes SIV, HTLV-I oder HTLV-II stammt und ein Epitop B enthält, und eine zweite Nucleotidsequenz, die ein Epitop T codiert, welches von einem Protein stammt, das nicht ein Hüllglykoprotein von den Retroviren HIV, pathogenes SIV, HTLV-I oder HTLV-II ist, gegebenenfalls unter der Kontrolle eines Promotors, der die Expression der ersten und zweiten Sequenzen unter Bildung eines Hybridmoleküls erlaubt, das einem der Moleküle nach einem der Ansprüche 1 bis 20 entspricht, in einem Wirt, ausgewählt aus

25

Hefen, Viren, Bakterien oder eukaryontischen Zellen.

23. Mit einer rekombinanten DNA nach Anspruch 22 transformierter Wirt, dadurch gekennzeichnet, daß es sich um eine Hefe, ein Virus, beispielsweise ein Baculovirus, ein Pockenvirus, ein Adenovirus oder ein Herpesvirus, eine eukaryontische Zelle oder ein Bakterium handelt, z.B. E. coli.

24. Immunogenes Mittel, das zur Induktion der in vivo-Produktion von neutralisierenden und gegen eines der Retroviren HIV, pathogenes SIV, HTLV-I oder HTLV-II schützenden Antikörpern, insbesondere gegen ein für den Menschen pathogenes HIV-Retrovirus, fähig ist, dadurch gekennzeichnet, daß es als Wirkstoff ein Hybridmolekül nach einem der Ansprüche 1 bis 7 oder ein Gemisch von Hybridmolekülen nach einem der Ansprüche 19 bis 21 umfaßt, in Verbindung mit einem pharmazeutisch verträglichen Vehikel.

25. DNA- oder RNA-Nucleotidsequenzen, die eine der Peptidsequenzen nach einem der Ansprüche 1 bis 10 codieren, oder eine der Nucleotidsequenzen, die die Epitope B nach einem der Ansprüche 6, 7, 9 oder 10 codieren.

26. Mittel für die in vitro-Diagnose einer Infektion durch ein HIV-Retrovirus, dadurch gekennzeichnet, daß es eine oder mehrere Nucleotidsequenzen nach Anspruch 25 oder ein Hybridmolekül nach einem der Ansprüche 1 bis 18 umfaßt.